# EUROPEAN PATENT APPLICATION

(11) **EP 4 046 756 A1**
(43) Date of publication of application: **24.08.2022**
(21) Application number: 19949064.0
(22) Date of filing: 17.10.2019
(51) Int. Cl.: B25J 9/06, A61B 8/00

(54) **MULTI-JOINT ROBOT**

(71) Applicant: Fuji Corporation, Chiryu-shi, Aichi 472-8686 (JP)
(72) Inventor: INAGAKI, Shigeyoshi, Chiryu-shi, Aichi 472-8686 (JP); YOSHIDA, Naofumi, Chiryu-shi, Aichi 472-8686 (JP); YAMASHITA, Yasuhiro, Chiryu-shi, Aichi 472-8686 (JP)
(74) Representative: Grünecker Patent- und Rechtsanwälte PartG mbB
(86) International application number: PCT/JP2019/040938
(87) International publication number: WO 2021/075031

(57) **Abstract**

A multi-joint robot includes: a base; a first arm connected to the base via a first joint shaft and configured to pivot in a horizontal direction with respect to the base; a second arm connected to the first arm via a second joint shaft and configured to pivot in a horizontal direction with respect to the first arm; a first rotation driving device configured to rotationally drive the first joint shaft; a second rotation driving device configured to rotationally drive the second joint shaft; a moving device configured to move the base in an axial direction of the first joint shaft; and a control device configured to control the first driving device, the second driving device, and the moving device.

## Description

### Technical Field

The present specification discloses a multi-joint robot.

### Background Art

Conventionally, there has been proposed a horizontal multi-joint robot including a base; a first arm connected to the base and turning around a first turning shaft along a vertical direction; a second arm connected to the first arm and turning around a second turning shaft along a vertical direction; and a third arm (lifting and lowering shaft) connected to the second arm and turning around a third turning shaft along a vertical direction and moving in an axial direction (vertical direction) of the third turning shaft (for example, refer to Patent Literature 1).

### Patent Literature

Patent Literature 1: JP-A-2019-130609

### Summary of the Invention

### Technical Problem

Incidentally, in the horizontal multi-joint robot described in Patent Literature 1, since the third arm serving as the lifting and lowering shaft is provided at a distal end of the second arm, the distal end portion of the second arm may be vertically increased in size. Therefore, such a horizontal multi-joint robot is not suitable for work performed in a narrow work space.

A main object of the present disclosure is to provide a multi-joint robot capable of easily performing work in a narrow work space.

### Solution to Problem

The present disclosure employs the following means in order to achieve the above-described main object.

It is the gist that a multi-joint robot of the present disclosure includes: a base; a first arm connected to the base via a first joint shaft and configured to pivot in a horizontal direction with respect to the base; a second arm connected to the first arm via a second joint shaft and configured to pivot in a horizontal direction with respect to the first arm; a first rotation driving device configured to rotationally drive the first joint shaft; a second rotation driving device configured to rotationally drive the second joint shaft; a moving device configured to move the base in an axial direction of the first joint shaft; and a control device configured to control the first driving device, the second driving device, and the moving device.

The multi-joint robot of the present disclosure includes the base, the first arm capable of horizontally pivoting with respect to the base and having the first joint shaft, and the second arm capable of horizontally pivoting with respect to the first arm and having the second joint shaft. In addition, the multi-joint robot includes the first driving device that rotationally drives the first joint shaft, the second driving device that rotationally drives the second joint shaft, and the moving device that moves the base in an axial direction of the first joint shaft. With this, a distal end portion of the arm can be made more compact as compared with a multi-joint robot including a lifting and lowering shaft provided at the distal end of the arm. As a result, it is possible to provide a multi-joint robot capable of easily performing work in a narrow work space.

### Brief Description of Drawings

Fig. 1 is a perspective view of an outer appearance of robot system 10 of the present embodiment.
Fig. 2 is a side view of robot system 10.
Fig. 3 is a block diagram showing an electrical connection relationship between robot 20 and control device 70.
Figs. 4A to 4C are views illustrating a state in which an orientation of rotary three-axis mechanism 50 is held by orientation holding device 37.
Fig. 5 is a view illustrating two operation modes of first arm 21 and second arm 22.
Figs. 6A and 6B are views illustrating a state in which the operation mode is switched due to interference object M.

### Description of Embodiments

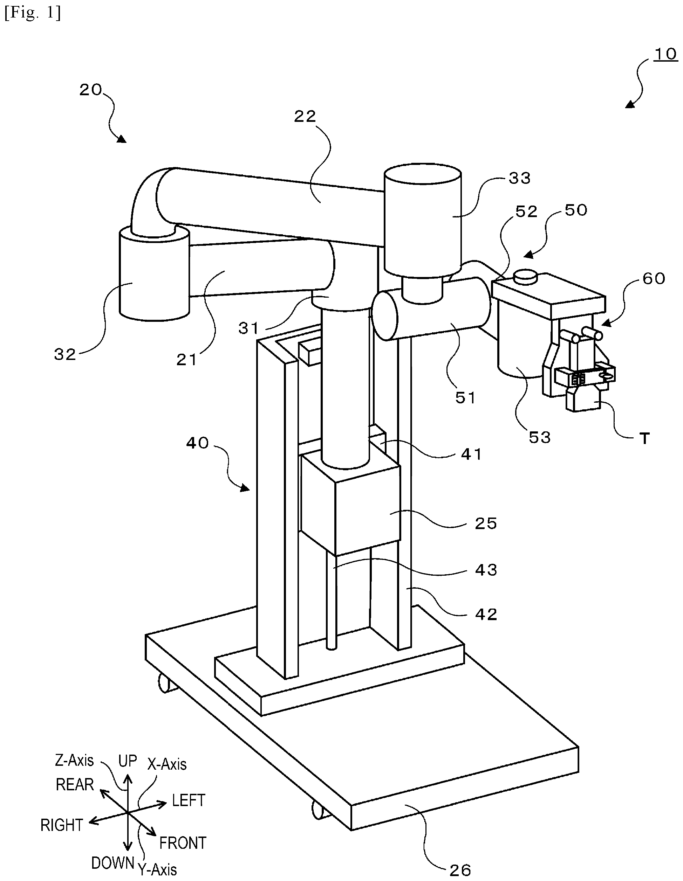

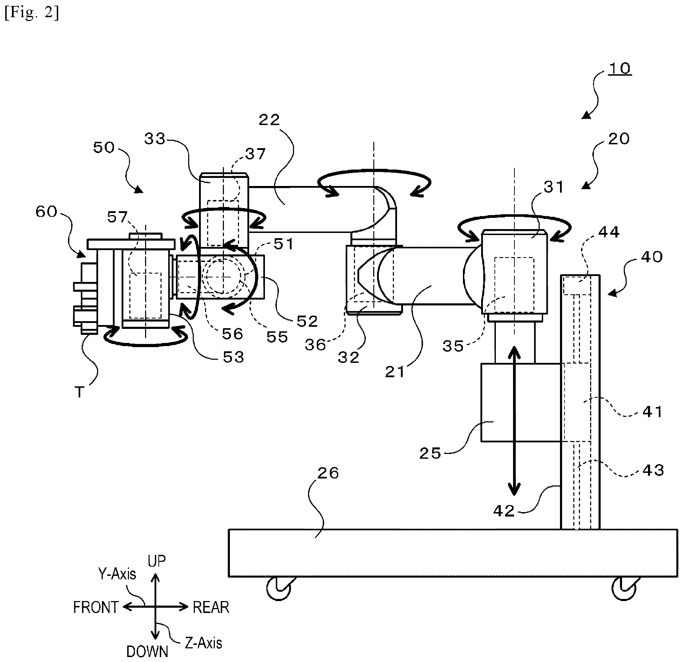

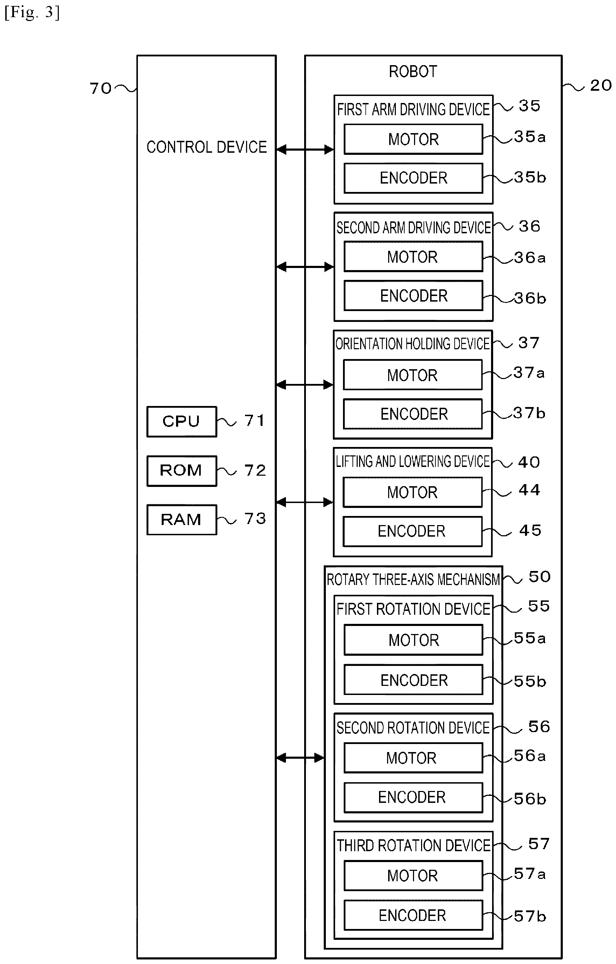

Next, an embodiment of the present disclosure will be described with reference to the drawings. Fig. 1 is a perspective view of an outer appearance of robot system 10 of the present embodiment. Fig. 2 is a side view of robot system 10. Fig. 3 is a block diagram illustrating an electrical connection relationship between robot 20 and control device 70. In Fig. 1, a right-left direction is an X-axis direction, a front-rear direction is a Y-axis direction, and an up-down direction is a Z-axis direction.

Robot system 10 of the embodiment performs predetermined work using tool T. Examples of the predetermined work may include work in which an ultrasonic probe is held by an arm and the arm is driven so that the ultrasonic probe is pressed against the skin of a subject upon ultrasound diagnosis. As shown in Figs. 1 and 2, robot system 10 includes robot 20 and control device 70 (refer to Fig. 3) that controls robot 20.

Robot 20 includes first arm 21, second arm 22, base 25, base stand 26, first arm driving device 35, second arm driving device 36, orientation holding device 37, lifting and lowering device 40, rotary three-axis mechanism 50, and tool holder 60.

A base end portion of first arm 21 is connected to base 25 via first joint shaft 31 extending in the up-down direction (the Z-axis direction). First arm driving device 35 includes motor 35a and encoder 35b. A rotary shaft of motor 35a is connected to first joint shaft 31 via a speed reducer (not shown). First arm driving device 35 makes first arm 21 turn (pivot) along a horizontal plane (XY-plane) around first joint shaft 31 as a fulcrum by rotationally driving first joint shaft 31 by motor 35a. Encoder 35b is attached to the rotary shaft of motor 35a, and is configured as a rotary encoder that detects a rotational displacement amount of motor 35a.

A base end portion of second arm 22 is connected to a distal end portion of first arm 21 via second joint shaft 32 extending in the up-down direction. Second arm driving device 36 includes motor 36a and encoder 36b. A rotary shaft of motor 36a is connected to second joint shaft 32 via a speed reducer (not shown). Second arm driving device 36 makes second arm 22 turn (pivot) along a horizontal plane around second joint shaft 32 as a fulcrum by rotationally driving second joint shaft 32 by motor 36a. Encoder 36b is attached to the rotary shaft of motor 36a, and is configured as a rotary encoder that detects the rotational displacement amount of motor 36a.

Base 25 is provided so as to be liftable and lowerable with respect to base stand 26 by lifting and lowering device 40 installed on base stand 26. As shown in Figs. 1 and 2, lifting and lowering device 40 includes slider 41 fixed to base 25, guide member 42 fixed to base stand 26 and extending in the up-down direction to guide the movement of slider 41, ball screw shaft 43 (lifting and lowering shaft) extending in the up-down direction and screwed with a ball screw nut (not shown) fixed to slider 41, motor 44 rotationally driving ball screw shaft 43, and encoder 45 (refer to Fig. 3). Lifting and lowering device 40 makes base 25 fixed to slider 41 move in the up-down direction along with guide member 42 by rotationally driving ball screw shaft 43 by motor 44. Encoder 45 is configured as a linear encoder that detects a position in the up-down direction (a lifting and lowering position) of slider 41 (base 25).

Rotary three-axis mechanism 50 is connected to a distal end portion of second arm 22 via orientation holding shaft 33 extending in the up-down direction. Rotary three-axis mechanism 50 includes first rotary shaft 51, second rotary shaft 52, and third rotary shaft 53 orthogonal to each other. First rotary shaft 51 is supported in an orientation orthogonal to orientation holding shaft 33. Second rotary shaft 52 is supported in an orientation orthogonal to first rotary shaft 51. Third rotary shaft 53 is supported in an orientation orthogonal to second rotary shaft 52. Tool holder 60 is attached to third rotary shaft 53. In the present embodiment, tool holder 60 is fixed at a position radially spaced apart from third rotary shaft 53. Tool T held by tool holder 60 moves along an arc-shaped locus centered on third rotary shaft 53 by the rotation of third rotary shaft 53. It should be noted that tool holder 60 may be attached such that tool T is positioned coaxially with third rotary shaft 53.

Orientation holding device 37 holds the orientation of rotary three-axis mechanism 50 (the direction of first rotary shaft 51) in a fixed direction regardless of the orientations of first arm 21 and second arm 22. Orientation holding device 37 includes motor 37a and encoder 37b. A rotary shaft of motor 37a is connected to orientation holding shaft 33 via a speed reducer (not shown).

Control device 70 is configured as a microprocessor centered on CPU 71, and includes ROM 72 or RAM 73, and an input/output port (not shown) in addition to CPU 71. A detection signal from each of encoders 35b, 36b, 37b, 45, 55b, 56b, and 57b is input to control device 70 via the input port. In addition, a drive signal to each of motors 35a, 36a, 37a, 44, 55a, 56a, and 57a is output from control device 70 via the output port.

In robot system 10 of the present embodiment configured as described above, tool T can be moved to any position in any orientation with a combination of the translational movement in three directions of the X-axis direction, the Y-axis direction, and the Z-axis direction by first arm driving device 35, second arm driving device 36, and lifting and lowering device 40, and the rotational movement in three directions, around the X-axis (pitching), around the Y-axis (rolling), and around the Z-axis (yawing), by rotary three-axis mechanism 50. With this, robot system 10 can perform necessary work using tool T. Specifically, control device 70 (CPU 71) determines a target position and a target orientation of an arm holding tool T. Subsequently, control device 70 individually sets the target rotation angle of first joint shaft 31, the target rotation angle of second joint shaft 32, the target rotation angle of orientation holding shaft 33, the target lifting and lowering position of base 25, the target rotation angle of first rotary shaft 51, the target rotation angle of second rotary shaft 52, and the target rotation angle of third rotary shaft 53, in order to move tool T to the target position in the target orientation. Control device 70 controls the corresponding motor so that the rotation angle or the lifting and lowering position detected by encoders 35b, 36b, 37b, 45, 55b, 56b, and 57b matches the corresponding target rotation angle or the corresponding target lifting and lowering position.

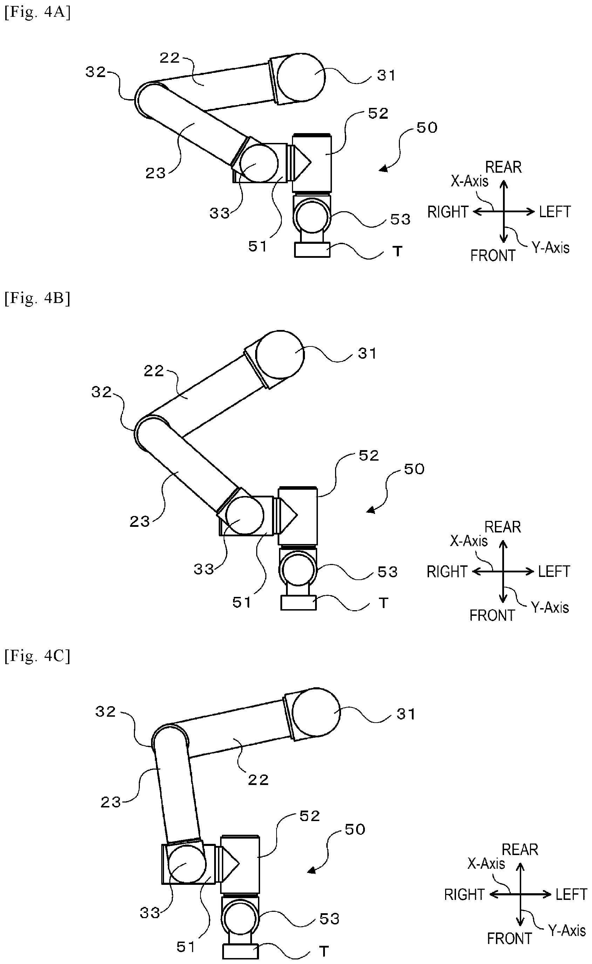

Here, as shown in Fig. 4, control device 70 sets the target rotation angle of orientation holding shaft 33 that supports first rotary shaft 51 so that the axial direction of first rotary shaft 51 is always in the right-left direction (the X-axis direction) regardless of the orientations of first arm 21 and second arm 22, and controls motor 44 so that the rotation angle detected by encoder 45 matches the set target rotation angle. The target rotation angle of orientation holding shaft 33 can be set based on the rotation angle of first joint shaft 31 detected by encoder 35b and the rotation angle of second joint shaft 32 detected by encoder 36b. With this, CPU 71 can independently perform the control of the translational movement in the three directions and the control of the rotational movement in the three directions described above, so that the control is facilitated.

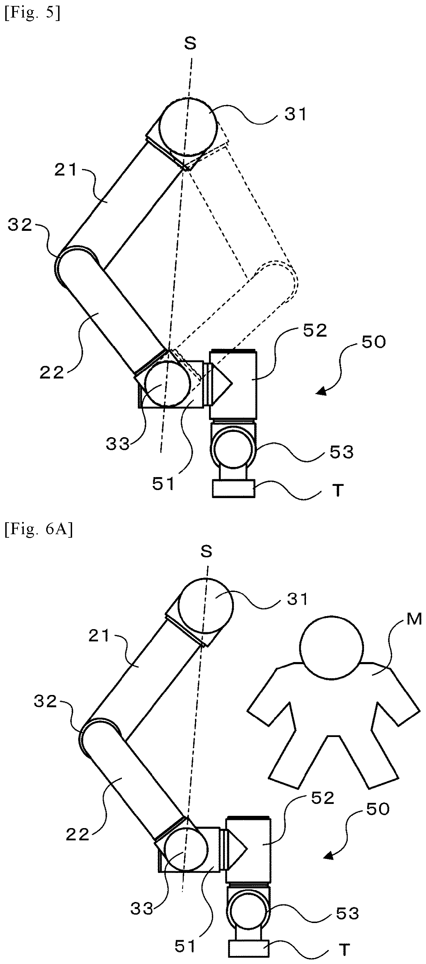

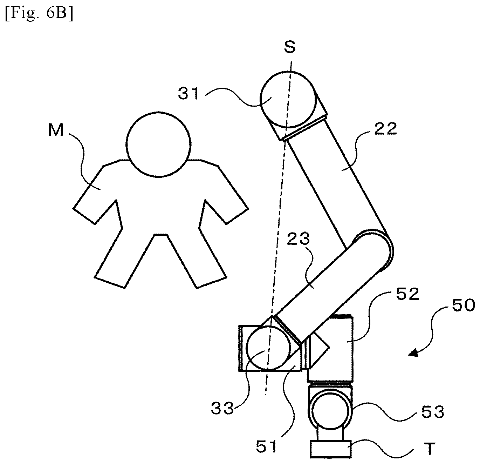

In addition, in robot system 10 of the present embodiment, first joint shaft 31 and second joint shaft 32 extend in the up-down direction, and support first arm 21 and second arm 22 turnably in the horizontal plane, respectively. Further, orientation holding shaft 33 extends in the up-down direction, and supports rotary three-axis mechanism 50 turnably in the horizontal plane. Therefore, as shown in Fig. 5, upon positioning tool T, two orientations (two operation modes) that are in a mirror image relationship with each other with respect to plane S including first joint shaft 31 and orientation holding shaft 33 can be selected for first arm 21 and second arm 22. Accordingly, in the present embodiment, as shown in Fig. 6A, when interference object M is positioned on a right side of robot system 10, control device 70 can perform work while avoiding interference object M by selecting a first operation mode in which first arm 21 and second arm 22 pivot so that second joint shaft 32 is positioned on a left side of plane S including first joint shaft 31 and orientation holding shaft 33. Alternatively, as shown in Fig. 6B, when interference object M is positioned on the left side of robot system 10, control device 70 can perform work while avoiding interference object M by selecting a second operation mode in which first arm 21 and second arm 22 pivot so that second joint shaft 32 is positioned on the right side of plane S including first joint shaft 31 and orientation holding shaft 33. It should be noted that the operation mode may be determined in advance by an operator's selection, or robot system 10 may detect the presence or absence of interference object M using a sensor to determine the operation mode.

Here, the correspondence relationship between main elements of the embodiment and main elements of the present disclosure recited in the scope of claims will be described. That is, base 25 of the present embodiment corresponds to the base of the present disclosure, first arm 21 corresponds to the first arm, first joint shaft 31 corresponds to the first joint shaft, second arm 22 corresponds to the second arm, second joint shaft 32 corresponds to the second joint shaft, first arm driving device 35 corresponds to the first rotation driving device, second arm driving device 36 corresponds to the second rotation driving device, lifting and lowering device 40 corresponds to the moving device, and control device 70 corresponds to the control device. In addition, rotary three-axis mechanism 50 corresponds to the rotary three-axis mechanism. Further, orientation holding shaft 33 corresponds to the third joint shaft, and orientation holding device 37 corresponds to the third rotation driving device.

Note that, the present disclosure is not limited to the above-described embodiment, and it goes without saying that the present disclosure can be carried out in various aspects without departing from the technical scope of the present disclosure.

For example, in the above-described embodiment, rotary three-axis mechanism 50 is attached to the distal end portion of second arm 22 via orientation holding shaft 33 for orientation holding, but may be attached directly to the distal end portion of second arm 22.

In the above-described embodiment, tool holder 60 (tool T) is attached to the distal end portion of second arm 22 via rotary three-axis mechanism 50 and orientation holding shaft 33, but may be attached to orientation holding shaft 33 or may be attached to the distal end portion of second arm 22.

In the above-described embodiment, base 25 is lifted and lowered by lifting and lowering device 40 formed by the ball screw mechanism; however, the configuration is not limited to this, and may be lifted and lowered by, for example, a linear motor.

As described above, it is the gist that a multi-joint robot of the present disclosure includes: a base; a first arm connected to the base via a first joint shaft and configured to pivot in a horizontal direction with respect to the base; a second arm connected to the first arm via a second joint shaft and configured to pivot in a horizontal direction with respect to the first arm; a first rotation driving device configured to rotationally drive the first joint shaft; a second rotation driving device configured to rotationally drive the second joint shaft; a moving device configured to move the base in an axial direction of the first joint shaft; and a control device configured to control the first rotation driving device, the second rotation driving device, and the moving device.

The multi-joint robot of the present disclosure includes the base, the first arm capable of horizontally pivoting with respect to the base and having the first joint shaft, and the second arm capable of horizontally pivoting with respect to the first arm and having the second joint shaft. In addition, the multi-joint robot includes the first driving device that rotationally drives the first joint shaft, the second driving device that rotationally drives the second joint shaft, and the moving device that moves the base in an axial direction of the first joint shaft. With this, a distal end portion of the arm can be made more compact as compared with a multi-joint robot including a lifting and lowering shaft provided at the distal end of the arm. As a result, it is possible to provide a multi-joint robot capable of easily performing work in a narrow work space.

In such a multi-joint robot of the present disclosure, a rotary three-axis mechanism disposed at the distal end of the second arm may be provided. In this case, the rotary three-axis mechanism may be attached to the distal end of the second arm via a third joint shaft parallel to the first and second joint shafts, the multi-joint robot may further include a third rotation driving device that rotationally drives the third joint shaft, and the control device may control the third rotation driving device so that the rotary three-axis mechanism is held in a fixed orientation regardless of rotational positions of the first and second joint shafts. With this, it is possible to independently perform the control of the translational movement by the first rotation driving device, the second rotation driving device, and the moving device, and the control of the rotational movement by the rotary three-axis mechanism, so that the control is facilitated.

Further, in the multi-joint robot of the present disclosure, two operation modes may be provided in which orientations of the first and second arms have a mirror image relationship with each other with respect to a plane including the first joint shaft and a distal end of the second arm. In this case, the control device may select and execute an operation mode that does not interfere with an interference object out of the two operation modes. As a result, it is possible to perform work even in a case where the interference object is adjacent to the multi-joint robot.

Further, in the multi-joint robot of the present disclosure, the moving device may be a lifting and lowering device that lifts and lowers the base in the up-down direction.

### Industrial Applicability

The present disclosure can be applied to, for example, the manufacturing industry of a multi-joint robot.

### Reference Signs List

10: robot system, 20: robot, 21: first arm, 22: second arm, 25: base, 26: base stand, 31: first joint shaft, 32: second joint shaft, 33: third joint shaft, 35: first arm driving device, 35a: motor, 35b: encoder, 36: second arm driving device, 36a: motor, 36b: encoder, 37: orientation holding device, 37a: motor, 37b: encoder, 40: lifting and lowering device, 41: slider, 42: guide member, 43: ball screw shaft, 44: motor, 45: encoder, 50: rotary three-axis mechanism, 51: first rotary shaft, 52: second rotary shaft, 53: third rotary shaft, 55: first rotation device, 55a: motor, 55b: encoder, 56: second rotation device, 56a: motor, 56b: encoder, 57: third rotation device, 57a: motor, 57b: encoder, 60: tool holder, 70: control device, 71: CPU, 72: ROM, 73: RAM, T: tool, M: interference object

## Claims

1. A multi-joint robot comprising:
a base;
a first arm connected to the base via a first joint shaft and configured to pivot in a horizontal direction with respect to the base;
a second arm connected to the first arm via a second joint shaft and configured to pivot in a horizontal direction with respect to the first arm;
a first rotation driving device configured to rotationally drive the first joint shaft;
a second rotation driving device configured to rotationally drive the second joint shaft;
a moving device configured to move the base in an axial direction of the first joint shaft; and
a control device configured to control the first driving device, the second driving device, and the moving device.

2. The multi-joint robot according to claim 1, further comprising:
a rotary three-axis mechanism disposed at a distal end of the second arm.

3. The multi-joint robot according to claim 2,
wherein the rotary three-axis mechanism is attached to the distal end of the second arm via a third joint shaft parallel to the first and second joint shafts,
the multi-joint robot further comprises a third rotation driving device configured to rotationally drive the third joint shaft, and
the control device controls the third rotation driving device so that the rotary three-axis mechanism is held in a fixed orientation regardless of rotational positions of the first and second joint shafts.

4. The multi-joint robot according to any one of claims 1 to 3,
wherein two operation modes are provided in which orientations of the first and second arms have a mirror image relationship with each other with respect to a plane including the first joint shaft and a distal end of the second arm.

5. The multi-joint robot according to claim 4,
wherein the control device selects and executes an operation mode that does not interfere with an interference object out of the two operation modes.

6. The multi-joint robot according to any one of claims 1 to 5,
wherein the moving device is a lifting and lowering device configured to lift and lower the base in an up-down direction.
